# EUROPEAN PATENT APPLICATION

(11) **EP 4 397 299 A1**
(43) Date of publication of application: **10.07.2024**
(21) Application number: 22862452.4
(22) Date of filing: 01.09.2022
(51) Int. Cl.: A61K 8/65, A61K 8/72, A61K 8/98

(54) **METHOD FOR PRODUCING PRESERVED COLLAGENOUS CONNECTIVE TISSUE, COLLAGENOUS CONNECTIVE TISSUE, USES THEREOF AND KIT FOR IMPLANT IN TISSUES**

(30) Priority: 02.09.2021 BR 102021017465
(71) Applicant: Labcor Laboratórios Ltda., 30431262 Belo Horizonte/MG (BR)
(72) Inventor: JOVIANO CASAGRANDE, Ivan Sérgio, 30350-710 Belo Horizonte (BR); PADILHA JUNQUEIRA DE SOUZA, Gustavo, 22411-003 Rio de Janeiro (BR)
(74) Representative: Herzog IP Patentanwalts GmbH
(86) International application number: PCT/BR2022/050348
(87) International publication number: WO 2023/028681

(57) **Abstract**

The present invention relates to the preservation of collagenous connective tissue so as to achieve the structural integrality of the collagen fibers, rendering the tissue suitable for use in implants without causing immune and/or inflammatory rejection, comprising the steps of trimming the collagenous connective tissue in a buffer solution, washing the connective tissue with the buffer solution, stabilizing the tissue in an ethanol solution, treating the tissue with polyethylene glycol solution, washing and storing the tissue in ethanol, sterilizing the tissue with an ethanol and hydrogen peroxide solution and storing the tissue for transport in ethanol and indomethacin solution. The preferred use of the present invention is in surgical interventions for correcting various anomalies of the human body, where the graft is particularly required.

## Description

### FIELD OF THE INVENTION

The present invention is focused on a method for producing collagen connective tissue, to the tissue thus produced and to the uses thereof. In particular, the present invention refers to the preservation of collagen connective tissue so as to provide the structural integrality of the collagen fibers, making said tissue suitable for used in implants without any immune and/or inflammatory rejection. The present invention is preferably utilized in surgical interventions to correct various anomalies of the human body, where a graft is particularly required.

### BACKGROUND OF THE INVENTION

In the domain of grafts or implantation, there are some examples in the state of the art using collagen connective tissue. For example, the patent US 9,205,172 B2 discloses a method of treating a biomaterial containing collagen to reduce or alleviate calcification and improve the longevity of the biomaterial, which may be used as an implantable device and methods for producing same. Patent BRPI0814523-7 B1 discloses a biocompatible composite implant and a method for forming same, comprising a framework of cross-linked tissue collagen processed without glutaraldehyde and an elongated member, wherein these are kept in contact with one another throughout the length of the framework.

Nevertheless, in the field of medicine there is still the need for an improved method for processing collagen connective tissue from the animal donor source, such that the collagen fibers can be implanted in a receptor without any immune and/or inflammatory rejection reaction, this being the main objective of the present invention.

Although the present invention does not have limited use to be used in reconstructive surgical procedures in human beings, below we will illustrate two applications of conventional grafts in surgical procedure: the first for (i) correction of penile curvature and the second in (ii) applications of facial reconstruction in human beings.

### (i) Penile curvature:

As well known in the fields of medicine, the penis, the male sexual organ, when in erect state, in most men, show some degree of curvature. Diversions less than 20 degrees on the axis of the penis are considered normal and in general do not adversely affect, for example, penetration, thus not causing problems for a man's sex life. However, diversions greater than 20 degrees are considered pathological and affect at least 10% of men around the world. Depending on the degree of the curvature, penetration may become difficult, or even impossible. An exaggerated penile curvature may be congenital in origin or be caused by Peyronie's Disease. Although this condition is benign and does not entail health risks for the patient, it may cause serious problems to a patient's sex life and psychology.

The penis is an organ that has the capacity to increase in size and become erectile when the man is sexually aroused. This is possible due to its anatomical structure, formed in the center by a bundle of tissue called spongy body, inside of which the urethra passes, and on the sides by two bundles of tissue called cavernous bodies. These tissues have internal spaces that fill with blood when a man is sexually aroused, making them increase in size and become more rigid, such as to enable penetration and, consequently, the sexual act. These tissues are coated externally by a compliant tissue, called tunica albuginea.

Peyronie's Disease is most common in men over 40 years old and is generally caused by micro-traumas in the tunica albuginea that may occur during the course of a patient's sex life. In the healing process of these micro-traumas, fibrous tissue may develop in the tunica albuginea, in the form of plaques or nodules that reduce the compliance of the tunica in the fibrosis area. Due to this loss of compliance in the area of fibrosis, when the penis becomes erect, the cavernous body cannot fully distend and the penis becomes curved.

For years, surgeons specialized in treating this problem have been trying to develop various techniques with a view to remedying the problem. However, most of them have had a low rate of success.

One of the techniques utilized today is the so-called "plicature", which consists of surgically reducing the side not affected by fibrosis, yet this procedure causes a reduction in size of the penis, which is an undesirable effect for most men.

Another common technique consists of surgically removing the fibrous tissue and replace it with some type of tissue graft, which may be of the autologous or self-graft type, where transplant occurs of the tissue of a part of the body to another of the same individual; (b) isograft, alograft or homologous graft, where transplant occurs of the tissue of the body of another animal of the same species, but with a different genotype to the individual; or (c) xenograft or heterologous graft, where transplant occurs of the tissue of the body between animals of different species.

If successful, these techniques do not reduce the size of the penis, being preferred by most patients. However, the tissue utilized for the graft must have some special characteristics, which are hard to find, chiefly in the heterologous tissues available on the market. Among these characteristics, the main ones are: the tissue utilized for the graft must have similar compliance to that of the tunica albuginea, otherwise, the problem will not be solved; the tissue utilized for the graft must be biocompatible and not cause allergic, inflammatory reactions or rejection; the tissue utilized for the graft not suffer from calcification; the tissue utilized for the graft must be capable of enabling growth of the cells of the very patient thereover, promoting biointegration; and tissue utilized for the graft must not sustain retraction after surgery, because otherwise the problem will not be solved.

All these characteristics are not present in the heterologous grafts available commercially, which justifies, at least in part, the low rate of success obtained today with this type of procedure.

One of the types of heterologous graft most commonly available on the market is the glutaraldehyde-preserved bovine pericardium graft. This type of graft has been utilized with relative success in various types of surgeries, chiefly vascular surgeries. However, attempts to use this in surgeries to correct penile curvature have not been successful. The main reasons for the lack of success of this type of graft are related to the fact that the glutaraldehyde-preserved bovine pericardium graft:
- does not have the compliance and flexibility necessary to adapt to the changes in size occurring in the penis;
- it usually presents retraction after surgery, causing the shortening of the member in the area repaired and leading to the failure of the procedure;
- it does not enable the patient's cells to grow on its surface, behaving like an alien body to the organism;
- although biocompatible, it tends to cause inflammatory reactions and undergo calcification, which leads to tissue hardening and, consequently, to the failure of the procedure.

One exemplary attempt at solving the problems cited above is patent US 7,008,763 B2, incorporated herein as reference in its totality. This document discloses the method for processing the collagen connective tissue with the structure of collagen fibers of a donor animal source, such that after the implant of said tissue in a receptor, said tissue contains an anti-inflammatory agent and is acceptable for the receptor without an immune rejection.

The method disclosed by US 7,008,763 B2 is based on the use of a glutaraldehyde-free preservative solution comprising a saline solution, a stabilizing solution, a solution composed of polyglycol, a salt, a phosphate buffer and an oxidizing agent.

However, this solution is not yet sufficient to achieve the tissue suitably preserved to the extent that it can be accepted by the human body since this solution did not prove effective and sufficient to achieve a tissue suitably preserved to the extent that it is accepted by the human body as tissue for albuginea membrane plasty in correcting penile curvature, for example.

Therefore, as stated above, there is still the need for an improved method for processing the collagen connective tissue from the animal donor source, such that the collagen fibers can be implanted in the receptor without any immune and/or inflammatory rejection reaction, this being the main objective of the present invention.

### (ii) Facial reconstruction:

The various techniques of oral and maxillofacial reconstruction techniques currently in practice include mucoperiosteal patches, bone replacement by way of grafts, guided bone regeneration and guided tissue regeneration with the use of bone replacements and barriers, such as absorbable and non-absorbable membranes.

Although there is no consensus on the ideal material for grafts, some important criteria guide the choice. The material must be easy to obtain, available in sufficient quantity, adaptable and capable of resisting infection and reabsorption. Additionally, it must be inert, resistant, easy to sterilize and not impede the function of other structures.

Scientific literature demonstrates that the autogenous cartilage is generally successful due to its low metabolism and absence of an adverse reaction. However, most autogenous grafts present limitations, such as dislodging, hardship of handling and delineating the graft contour, rate of reabsorption and retraction, besides donor site morbidity.

So that the use of the biomaterial is successful, same must be biotolerated, that is, not cause local or systemic damage, not be toxic, carcinogenic or radioactive. Additionally, an ideal biomaterial must be manufacturable, sterilizable and stable during its application or implantation. Studies report that the use of bioabsorbable materials has advantages in relation to non-absorbable ones, since they avoid problems of migration, extrusion and tardy infection.

The biomaterials most commonly used in BMF surgery are the absorbable collagen membranes. Despite their good performance, they may display certain limitations in specific cases, such as thickness, resistance to tension, volume and size being less than expected. In these cases, the use of autogenous myofascial patches is often resorted to.

It is thus desirable to provide thicker biomaterial that is greater in size and resistance to tension so that it can be advantageously utilized in the facial reconstruction so as to avoid the problems referred to above and provide results not yet achieved by using other materials from the state of the art.

### DESCRIPTION OF THE INVENTION

The method for producing preserved collagen connective tissue from an animal donor source according to the present invention comprises the following steps:
a) after removal from the donor, trim the collagen connective tissue in a buffer solution;
b) wash the connective tissue with the same buffer solution
c) c) stabilize the tissue with an ethanol solution 50%;
d) d) treat the tissue with the solution of polyethylene glycol;
e) e) wash and conserve the tissue in ethanol 50%;
f) sterilize the tissue with an ethanol solution 50% and hydrogen peroxide (H₂O₂) 1.5%; and
g) store the tissue in ethanol solution 50% and indomethacin.

More specifically, the buffer solution according to the present invention is a mixture of phosphate buffer solution, composed of monobasic sodium phosphate (NaH₂PO₄) 0.1% and dibasic sodium phosphate (Na₂HPO₄) 0.6% and solution of sodium chloride (NaCl) 0.9%, and the pH of the solution is physiological, preferably in the range of 7.3 to 7.5 and the temperature must be comprised in a range from 5ºC to 15°C. Further preferably, about 500 ml of the buffer solution is used to wash each piece of collagen connective tissue, about 56 grams per liter of buffer solution being used.

The stabilization of the collagen connective tissue must be carried out with ethanol 50% at a temperature in the range between 2°C and 10°C.

The stabilized tissue is then treated with a solution of polyethylene glycol comprising the mixture of polyethylene glycol 6% by volume, sodium chloride (NaCl) 32% by volume, phosphate buffer solution 13% by volume, and hydrogen peroxide (H₂O₂) 2% by volume, the temperature being controlled within the range from 2°C to 8°C.

The tissue thus stabilized is then washed and conserved in a solution of ethanol 50% at a temperature of around 25°C, being subsequently sterilized with a solution of ethanol 50% and hydrogen peroxide (H₂O₂) 1.5%.

Finally, the collagen connective tissue is stored ready for use in a solution of ethanol 50% by volume and indometacin about 0.05% by volume for a period of up to 2 years.

The washing steps with the buffer solution, stabilization with ethanol solution 50% and washing and conservation of the collagen connective tissue with ethanol solution 50% are advantageously repeated from 3 to 6 times, and preferably the washing step of the tissue is repeated for 5 the 6 times, the stabilization is repeated for 3 times and the washing step and conservation is repeated from 3 to 5 times.

Additionally, in the steps of stabilization, treatment and sterilization of the collagen connective tissue, the immersion time varies from 24 to 96 hours, the stabilization step preferably being carried out for 24 hours for each repetition, the treatment step for 96 hours and the sterilization step for 24 hours.

### EXAMPLES OF APPLICATION OF THE INVENTION

### Penile Curvature:

The following example will describe an evaluation test program of the graft implant comprising an aldehyde-free bovine pericardium according to the present invention, in procedures of correction of penile curvature, a subjective evaluation of the satisfaction of the patients being reported with the correction made.

A total of 15 individuals were operated on, of which 13 had Peyronie's Disease and 2 had cogenital penile curvature. The surgeries were carried out between April/2019 and December/2020 at the Rio de Janeiro State University - Medical Sciences Faculty of the Pedro Ernesto University Hospital - Urology Department. The age of the patients ranged from 18 to 72 years old. Among the patients with Peyronie's Disease, 3 had been previously submitted to associated semi-rigid penile prosthesis implants.

Among the survey individuals, 11 had already completed the post-operatory monitoring period of 12 months, following the survey protocol. Among these, 7 were re-evaluated regarding satisfaction with the surgery, correction of the curvature, presence of surgical complications, quality of the erection and penile length.

A scale of 1 to 5 was used for subjective evaluation of the satisfaction of the individuals with the correction of the curvature. Four patients (57%) stated they were satisfied (4/5 in the scale) and three patients (43%) very satisfied (5/5 in the scale). Satisfactory correction of the penile curvature was obtained in 89% of the cases and just one patient, who presented complex penile curvature in 2 axes, still had a significant penile curvature in one of the axes (approximately 60º). Notwithstanding, this patient does not report difficulties in having penetrative sex. No significant complication was identified in the 7 patients reevaluated thus far, according to the Clavien Dindo scale. One individual surveyed showed a poorer erection quality after the procedure (11 %), evaluated by the International Index of Erectile Function (IIEF). There was an increase in the penile length in 6 of the 7 patients evaluated (86%) and this increased varied from 0.7 to 3.0 cm. A patient evolved with a reduction of the penile length by 0.5 cm.

In the preliminary security analysis involving 46% of the estimated total sample, it can be concluded that the patients were subjectively satisfied, with acceptable rates of success in the corrections of the penile curvatures and without complications that prevent, for the time being, the resumption of the study of the aldehyde-free pericardium graft implants in patients with penile curvature due to Peyronie's Disease or congenital curvature.

Besides the excellent results obtained with the method for producing preserved collagen connective tissue from an animal donor source according to the present invention, it was possible to note, in addition to the reduction of the anti-inflammatory reaction, other unexpected characteristics, such as, for example:
- the graft enables the growth of structural cells of the patient itself over same, promoting biointegration in such a way that the graft begins to display functional and biological characteristics similar to the tissue on which it was grafted, such as vascularization, enervation and incorporation of collagen cells and elastin, to the point that after some time it is no longer possible to differentiate the grafted tissue from the native tissue;
- the tissue presents elasticity, flexibility and compliance, even enabling the growth of the graft when necessary for adjusting the anatomical alterations sustained by the patient;
- differently to the graft of the state of the art treated with glutaraldehyde, the graft produced by the method of the present invention does not undergo retraction after the surgery.

### Facial reconstruction:

A study was carried out on facial reconstruction with the use of graft, the use of absorbable collagen biomaterial and aldehyde-free compounds, produced, preserved and stabilized according to the method described by the present invention, with nine patients over the age of 18, submitted to facial reconstruction surgeries at the Department of Odontology at the Santa Casa de Misericórdia Hospital in Poços de Caldas (MG), from March to November of 2021. This study was submitted to and approved by the ethics committee of said institution. All the patients acknowledged and signed the term of free consent.

The graft investigated was an aldehyde-free bovine pericardium membrane manufactured by the company Labcor Laboratórios Ltda. (Contagem-MG), produced by the method described by the present invention. The product was manufactured with non-aldehydic technology.

### • Case 1:

A first study involving the preserved non-aldehydic collagen connective tissue was carried out with a patient of the female sex, aged 36, with a history of ankylosis in temporomandibular joint (ATM) left (L) associated to dentofacial deformity. The patient had been submitted to three surgeries, among which one procedure for correcting bone distraction in mandible and other for release of the ATM E with implantation of silicon prosthesis and ostectomy of the head of the mandible E. The patient had scars from prior surgeries, severe limitation of the mouth opening, terrible state of oral hygiene with multiple caries, advanced periodontal disease, teeth withheld in the mandible and cystic-like damages in the mandible and maxilla. He also had an infectious condition an abscess on the E side, where the prosthesis was exposed.

The patient underwent surgery under general anesthesia for removal of all teeth in the mandible and maxilla and installation of implants in maxilla and mandible in the *all-on-foursystem.* After removal of the damages in the maxilla and mandible and installation of the implants, the empty spaces were filled with biomaterial. Externally, an Al Kayat access was made. The prosthesis was extracted and the tissues originating from the chronic inflammatory process were removed, exposing the joint bone surfaces. The aldehyde-free bovine pericardium membrane graft was installed as if it were a temporal patch, placed between the remaining head of the mandible and the glenoid cavity, and was fastened under tension with absorbable yarn.

The pre-auricular incision was made, partially accompanying the fistula existing in the region. Planar divulsion required the utmost caution due to the changes in the local anatomy and alterations in the local tissues by the persistence of an old infectious/inflammatory process, besides the presence of a silicon orthosis which was interposed between the tissues, from the temporomandibular joint E up to the auricular region. Upon accessing the joint through the "path" left by the reaction to the silicon orthosis, we found tissue partially fibrous and partially granulomatous in the cavity of the former ATM E. This material acted as a barrier between the remainder of the head of the mandible E and the glenoid cavity (mandibular fossa).

With the removal of the orthosis, a mechanical cleaning of the site was necessary, which exposed the joint bone surfaces or the remainder thereof. In order to avoid a local inflammatory and/or restorative process, with consequent loss of movement on that side, we considered using an E temporal myofascial patch. However, the local fibrosis and the characteristics modified by the chronic infectious process made a satisfactory patch impossible. For this reason, the aldehyde-free bovine pericardium membrane graft was used due to the characteristics similar to a patch of this nature, besides the resistance and capacity to adapt to the region, and can be fastened by local sutures.

The aldehyde-free bovine pericardium membrane graft was employed for interposition between the bone surfaces and closing of the fistula. Said graft was sutured to the adjacent tissues under constant tension so it remained immobile. The post-operative tomography indicated satisfactory position of the graft and one week after the surgical procedure, the patient showed an improvement in mouth opening and closing, previously limited. The patient did not show any inflammatory reaction or rejection to the graft, even after six months of monitoring, thus demonstrating the total feasibility of the use of the graft of preserved non-aldehydic collagen connective tissue for facial reconstruction.

### • Case 2:

A second study involving the preserved non-aldehydic collagen connective tissue was carried out with the female patient aged 69, with the history of multiple facial fractures for over twenty years at least, presence of silicone prosthesis dislodged from the floor of orbit and infectious process in the orbit E with fistula in the inner part of the lower eyelid E. The prosthesis produced pressure on the eyeball, generating diplopia and "blurred vision". The correction was carried out under general anesthesia with infraorbital access E. After withdrawing the prosthesis, the *bone gap* on the lower edge of orbit E was adjusted using a titanium miniplaque of about 1.5 mm, fastened by means of two screws about 1.5 mm in diameter. The preserved non-aldehydic collagen connective tissue graft was accommodated on the floor of the orbit to occupy the space resulting from the absence of the prosthesis (which occupied the region, sustaining the eye) and to correct the deformity of the orbit. The graft was fastened to the plaque and to the adjacent tissues, occluding the fistula.

The patient already had a silicon orthosis in face E which caused a fistula in the internal region of the lower eyelid, causing part of the orthosis to come out and compressing the eyeball. Besides the pain and diplopia reported by the patient, the chronic infectious/inflammatory process generated ongoing secretion in the eye E. The option was then taken to make an infraorbit E incision using the expression line below the eyelid so that access was broader and in order to preserve the eyelid, enabling the withdrawal of the orthosis. After the withdrawal of the orthosis, we noted the dislodging of the eye E which invaded the area of the maxillary sinus, besides the bone space in the lower rim of the orbit E which enabled said dislodging. With caution, the tissues were divulsed and the bone remains from the floor of the orbit E were exposed, also limiting the space which originally pertained to the maxillary sinus E. A titanium plaque of about 1.5 mm was employed to redo the lower rim, since the entire bone structure was modified by decades since the facial trauma.

Even with the recovery of a lower orbital rim, same was below the right orbital rim (R). Then there was the need to interpose the material on the floor of the orbit to raise the eye and enable the closing of the fistula and the reconstruction of the eyelid. In the impossibility of using local patches and due to the need to avoid alloplastic materials due to the prior chronic process, the non-aldehydic collagen connective tissue was used as graft. This graft was sutured to the adjacent tissues and fastened to the plaque with absorbable yarns. The graft was folded to produce volume and raise the eye E. Part of the graft was sutured to the internal tissues of the eyelid E, closing the fistula and partially rebuilding it. After monitoring for six months, the patient showed an improvement of the diplopia, correction of the fistula and repositioning of the eyeball.

### • Case 3:

A third study involving the preserved non-aldehydic collagen connective tissue was carried out on the female patient aged 76, with periodontal damage in the region of the teeth 43, 44 and 45. Previously, the patient had undergone a curettage and biomaterial graft, as indicated by the imaging examinations. The examinations also revealed a fistula in the region of tooth 45 on the buccal side and an increase on the lingual side in the region of teeth 43, 44 and 45. On palpation, the region presented a hardened appearance.

The surgical approach was buccal, preserving the mentonian foramen and its structures. After removing the damage and the teeth referred to above, we noted the damage to the bottom with hardened appearance and closely associated to the salivary ducts of the region. The second damage was carefully removed without harming the ducts, preparing the region for the graft.

The preserved collagen connective tissue was sutured to the lingual tissues isolating the ducts. A bone graft was made with biomaterial and the preserved collagen connective tissue was folded over the graft to act as barrier. The preserved collagen connective tissue was sutured in tension on the graft.

The damage was diagnosed as ameloblastoma, being considered the reconstruction through guided bone regeneration (GBR) to enable visualization and removal of the damage. The preserved collagen connective tissue was used due to the large extent of the repair to be done and the need for an extensive barrier which is not available on the market. The choice of the preserved collagen connective tissue was also based on the possibility of handling and fastening the membrane to the local tissues. A slight exposure of the preserved collagen connective tissue was noted in the post-operatory period, which in no way compromised the results.

### • Case 4:

A fourth study involving the preserved non-aldehydic collagen connective tissue was carried out on the male patient aged 36, who had lost his two lower central incisors. In the surgery for graft at hand, the preserved collagen connective tissue was utilized as a barrier for the bone graft and as substitute for the connective tissue for improving the gingival recession in the lower incisors, so as to ensure the possibility of future rehabilitation with implants.

Medical literature indicates guided bone regeneration (GBR) and the use of connective grafts due to the thin gingival profile of the patient. Therefore, the case required a bone graft, a barrier for the graft and a connective tissue graft. Said demands, added to the great extent of the defect, guided the choice of the preserved collagen connective tissue, which could act as barrier and replacement of connective tissue, improving the gingival profile.

After the exhibition and preparation of the bone bed for graft and the implantation of the bone graft, the preserved collagen connective tissue was sutured to the buccal periosteum, folded over the bone graft and sutured to the tissues of the region lingual. The results two months after the procedure were altogether satisfactory. In the six-month monitoring period, there was total recovery of the alveolar rim and of the gingival profile.

### • Case 5:

A fifth study involving the preserved non-aldehydic collagen connective tissue was carried out on the female patient aged 32, indicated for rehabilitation with implant due to the loss of the tooth 44, with consequent buccal dehiscence.

The preserved collagen connective tissue on the buccal side was used for gingival profile gain, since the patient was opposed to removing connective tissue from a second surgical site. The preserved collagen connective tissue was employed as tissue graft and fastened by suture. One month after the procedure showed perfect correction.

### • Case 6:

A sixth study involving the preserved non-aldehydic collagen connective tissue was carried out on a female patient aged 62, with root fracture in tooth 34 and buccal bone loss. After removal of the residual root, an implant was placed at the site. The bone loss was offset by the biomaterial graft and the preserved collagen connective tissue acted as a barrier for preventing gingival recession.

An implant through immediate loading was installed in the region of the lower pre-molar E. Due to the buccal bone loss, the option for guided bone regeneration (GBR) was taken. However, the soft tissues were insufficient to coat the bone graft, as the root fracture and the chronic inflammatory process in the region had caused gingival recession. The option for preserved collagen connective tissue was taken, since this would serve as barrier for bone regeneration and would enable gingival tissue gain. The results after two months, with the prosthesis installed over the implant, were excellent.

### • Case 7:

A seventh study involving the preserved non-aldehydic collagen connective tissue was carried out on a female patient, aged 37, smoker, with poor oral hygiene conditions, presenting the destruction of various crowns of the remaining teeth and periodontal disease. Previously, she had been submitted to multiple teeth extractions, both in the mandible and maxilla, for installation of dental implants of the morse cone type with the intention of rehabilitation with protocol-type prostheses in both dental arches.

The loss of tissue gengival in the maxilla due to the removal of damages associated to the periodontal disease hampered the closing of the patch. A loss of volume in the maxilla was also noted. The option was taken to use the preserved collagen connective tissue (instead of the bone graft) in order to gain tissue in the vicinity of the implants, avoid possible recessions and provide buccal volume. The preserved collagen connective tissue was divided into a right fragment and another left, which were adapted to the prosthetic components (minipillars). Accordingly, the preserved collagen connective tissue was perforated in the points corresponding to the minipillars and adjusted at its bases, at the junction between the minipillars and the implants. The preserved collagen connective tissue was placed over the buccal bone tissue of the maxilla as if it were the periosteum. The suture was made with non-absorbable filament and taken out in 15 days.

Another reason for choosing the preserved collagen connective tissue was the need to remove of the surrounding areas of the patient's teeth, due to the periodontal disease and to smoking, which resulted in insufficient patch to cover the vicinity of the implants. The preserved collagen connective tissue also enabled gain in buccal tissue in the maxilla, improving the emergency profile of the prosthesis and posture of the upper lip.

### • Case 8:

An eighth study involving the preserved non-aldehydic collagen connective tissue was carried out on the male patient, aged 59, with damages in the mandible suggestive of mandibular cysts, which formed after tooth extractions for use of totally removable prosthesis.

The bone site at D revealed exposure of the lower alveolar plexus with damage anterior D. After surgical removal of the damages, the bone graft implant with biomaterial was made. The preserved collagen connective tissue was used as barrier in the GBR, having been sutured lingually and buccally so that it would be tensioned over the bone graft. Diagnosis: ceratocystis. The imaging examinations after the surgical procedure indicated good absorption and recovery of the patient. The result after six months was total recovery of the damages.

### • Case 9:

A ninth study involving the preserved non-aldehydic collagen connective tissue was carried out on the male patient, aged 44, who had complications from an orthognathic surgery. The patient had been referred by his orthodontist to have a tooth extraction of the element 47, which was compromised due to the post-operatory fracture of the first surgery. However, as a result of the infectious process and from the complications of the first surgical procedure, a fastening plaque was exposed in the region, which was removed in a second corrective surgery.

The tooth extraction of the tooth had its challenges, since the tissues of the region had no elasticity and there was intense fibrosis due to the prior infectious process. The preserved collagen connective tissue was employed in the removal of the tooth to assure that the exposure of the plaque did not get worse and so that there was no major reaction of the tissues, which would result in the exposure of the bone after the tooth extraction. The preserved collagen connective tissue was used to recover the remaining bone after the tooth extraction. The graft was sutured lingually, doubled over the exposed bone, and again sutured buccally keeping the tension over the covered area.

In this case, suture dehiscence was predicted due to the tissues having lost their elasticity. This in fact happened, exposing part of the preserved collagen connective tissue, which was kept by rinsing with 0.12% chlorhexidine.

As seen above, the preserved collagen connective tissue and stabilized can be utilized in various recovery facial applications, since being a preserved non-aldehydic biomaterial, it showed broad versatility and produced results beyond short and long-term expectations, also avoiding the extraction of autogenous patches in some situations.

These unexpected characteristics demonstrate that the preserved collagen connective tissue from an animal donor source, according to the present invention, is an excellent option for the use as graft in various types of surgeries, such as heart surgery, ophthalmological surgery, neurological surgery and surgery to correction penile curvature as reported above, among other applications where these unexpected characteristics can be indicated, since said connective tissue produced according to the present invention bears all the characteristics considered ideal for these purposes.

Additionally, the present invention also refers to the preserved collagen connective tissue by the method described above, its uses in reparation surgery with collagen connective tissue in any region or organ of the human body, as well as a surgical kit comprising the preserved collagen connective tissue and stabilized.

## Claims

1. **A METHOD FOR PRODUCING PRESERVED COLLAGENOUS CONNECTIVE TISSUE, characterized by** comprising the following steps:
a) after removal from the donor, trim the collagen connective tissue in a buffer solution;
b) wash the connective tissue with the buffer solution described in step a);
c) stabilize the tissue with an ethanol solution 50%;
d) treat the tissue with the solution of polyethylene glycol;
e) wash and conserve the tissue in ethanol 50%;
f) sterilize the tissue with an ethanol solution 50% and hydrogen peroxide (H₂O₂) 1.5%; and
g) store the tissue for transport in ethanol solution 50% and indomethacin.

2. The **METHOD FOR PRODUCING PRESERVED COLLAGENOUS CONNECTIVE TISSUE** according to claim 1, **characterized by** the buffer solution of step a) being the solution of phosphate buffer and sodium chloride (NaCl) 0.9%.

3. The **METHOD FOR PRODUCING PRESERVED COLLAGENOUS CONNECTIVE TISSUE** according to claim 2, **characterized by** the solution of phosphate buffer being a mixture composed of monobasic sodium phosphate (NaH₂PO₄) 0.1% by volume and dibasic sodium phosphate (Na₂HPO₄) 0.6% by volume.

4. The **METHOD FOR PRODUCING PRESERVED COLLAGENOUS CONNECTIVE TISSUE** according to claim 1, **characterized by** the buffer solution of step a) being at a temperature in the range of 5°C to 15°C.

5. The **METHOD FOR PRODUCING PRESERVED COLLAGENOUS CONNECTIVE TISSUE** according to claim 1, **characterized by** the pH of step a) being in the range of 7.3 to 7.5.

6. The **METHOD FOR PRODUCING PRESERVED COLLAGENOUS CONNECTIVE TISSUE** according to claim 1, **characterized by** the volume of buffer solution of step b) being 500 ml per piece of pericardium.

7. The **METHOD FOR PRODUCING PRESERVED COLLAGENOUS CONNECTIVE TISSUE** according to claim 1, **characterized by** the tissue stabilization temperature of step c) being in the range of 2°C to 10°C.

8. The **METHOD FOR PRODUCING PRESERVED COLLAGENOUS CONNECTIVE TISSUE** according to claim 1, **characterized by** the solution of polyethylene glycol of step d) being the mixture of polyethylene glycol 6% by volume, sodium chloride (NaCl) 32% by volume, phosphate buffer solution 13% by volume and hydrogen peroxide (H₂O₂) 2% by volume.

9. The **METHOD FOR PRODUCING PRESERVED COLLAGENOUS CONNECTIVE TISSUE,** according to either of claims 1 or 8, **characterized by** the tissue treatment temperature of step d) being in the range of 2°C to 8°C.

10. The **METHOD FOR PRODUCING PRESERVED COLLAGENOUS CONNECTIVE TISSUE** according to claim 1, **characterized by** the temperature of the ethanol solution 50% of step e) being around 25°C.

11. The **METHOD FOR PRODUCING PRESERVED COLLAGENOUS CONNECTIVE TISSUE** according to claim 1, **characterized by** the concentration of indometacin being about 0.05% by volume.

12. The **METHOD FOR PRODUCING PRESERVED COLLAGENOUS CONNECTIVE TISSUE** according to claim 1, **characterized by** the steps of washing, stability and cleanliness of the connective tissue being repeated from 3 to 6 times, preferably in step b) from 5 to 6 times, in step c) are repeated 3 times and in step e) from 3 to 5 times.

13. The **METHOD FOR PRODUCING PRESERVED COLLAGENOUS CONNECTIVE TISSUE** according to claim 1, **characterized by** the steps of treatment, stability and sterilization of the connective tissue being carried out in an immersion time of 24 to 96 hours, preferably in step c) 24 hours for each repetition, in step d) 96 hours and in step f) 24 hours.

14. The **COLLAGENOUS CONNECTIVE TISSUE, characterized by** comprising collagen connective tissue removed from a donor and being preserved and stabilized according to the method defined in any of claims 1 to 13.

15. The **COLLAGENOUS CONNECTIVE TISSUE,** as defined in claim 14, **characterized by** the tissue being the heterogeneous graft.

16. A **KIT FOR PRODUCING PRESERVED COLLAGENOUS CONNECTIVE TISSUE, characterized by** comprising a collagen connective tissue as defined in claim 14 and a solution of storage comprising a mixture of ethanol 50% by volume and indometacin about 0.05% by volume.

17. The **USE OF COLLAGENOUS CONNECTIVE TISSUE,** as defined in either of claims 14 or 15, **characterized by** being destined for application in any region or organ of the human body.

18. The **USE OF COLLAGENOUS CONNECTIVE TISSUE** according to claim 17, **characterized by** being destined for application as graft in heart surgery, ophthalmological surgery, neurological surgery and surgery for correcting parts of the human body.

19. The **USE OF COLLAGENOUS CONNECTIVE TISSUE,** as defined in claim 18, **characterized by** being destined for correcting penile curvature.

20. The **USE OF COLLAGENOUS CONNECTIVE TISSUE,** as defined in claim 18, **characterized by** being destined for facial reconstruction.

21. The **USE OF COLLAGENOUS CONNECTIVE TISSUE** according to claim 20, **characterized by** being for repairing ankylosis in the temporomandibular joint (TMJ).

22. The **USE OF COLLAGENOUS CONNECTIVE TISSUE** according to claim 20, **characterized by** being for repairing dentofacial deformities.

23. The **USE OF COLLAGENOUS CONNECTIVE TISSUE** according to claim 20, **characterized by** being for repairing deformities arising from bone fractures in the face.

24. The **USE OF COLLAGENOUS CONNECTIVE TISSUE** according to claim 20, **characterized by** being for suturing and reconstruction of the lingual tissues, as a form of barrier and replenisher.

25. The **USE OF COLLAGENOUS CONNECTIVE TISSUE** according to claim 24, **characterized by** the fact that the reconstruction is made through guided bone regeneration (GBR).

26. The **USE OF COLLAGENOUS CONNECTIVE TISSUE,** according to either of claims 20 or 23, **characterized by** being for bone lining in the face.

27. The **USE OF COLLAGENOUS CONNECTIVE TISSUE** according to claim 20, **characterized by** the fact of being for gaining buccal tissue in the jaws.
